# EUROPEAN PATENT SPECIFICATION

(11) **EP 1 870 449 B1**
(45) Date of publication and mention of the grant of the patent: **28.04.2010**
(21) Application number: 07101028.4
(22) Date of filing: 23.01.2007
(51) Int. Cl.: C12N 1/06, C12N 13/00, G01N 33/543

(54) **Method of concentrating and disrupting cells or viruses**
Verfahren zum Konzentrieren und Aufschluss von Zellen oder Viren
Procédé pour concentrer et briser les céllules ou les virus

(30) Priority: 07.04.2006 KR 20060031929
(43) Date of publication of application: 26.12.2007
(73) Proprietor: Samsung Electronics Co., Ltd., Suwon-si, Gyeonggi-do (KR)
(72) Inventor: Cho, Yoon-kyoung Samsung Advanced Institute of Technology, Gyeonggi-do (KR); Lee, Jeong-gun Samsung Advanced Institute of Technology, Gyeonggi-do (KR); Park, Jong-myeon Samsung Advanced Institute of Technology, Gyeonggi-do (KR); Lee, Young-sun Samsung Advanced Institute of Technology, Gyeonggi-do (KR); Lee, Hyo-yeon Samsung Advanced Institute of Technology, Gyeonggi-do (KR)
(74) Representative: Grünecker, Kinkeldey, Stockmair & Schwanhäusser Anwaltssozietät

(56) References cited:
- EP-A- 0 605 003
- EP-A- 1 650 297
- EP-A- 1 655 366
- EP-A- 1 662 008
- EP-A- 1 842 914
- PITSILLIDES COSTAS M ET AL: "Selective cell targeting with light-absorbing microparticles and nanoparticles." BIOPHYSICAL JOURNAL, vol. 84, no. 6, June 2003 (2003-06), pages 4023-4032, XP002428862 ISSN: 0006-3495
- HOFMANN O ET AL: "LASER BASED DISRUPTION OF BACILLUS SPORES ON A MICROCHIP" SPECIAL PUBLICATION - ROYAL SOCIETY OF CHEMISTRY, ROYAL SOCIETY OF CHEMISTRY, LONDON, GB, 2005, pages 258-260, XP009066976 ISSN: 0260-6291
- BECQUART ET AL: "Quantitation of HIV-1 RNA in breast milk by real time PCR" JOURNAL OF VIROLOGICAL METHODS, AMSTERDAM, NL, vol. 133, no. 1, 1 April 2006 (2006-04-01), pages 109-111, XP005333414 ISSN: 0166-0934 [retrieved on 2005-12-15]
- HUANG TOM T ET AL: "Micro-assembly of functionalized particulate monolayer on C18-derivatized SiO2 surfaces" BIOTECHNOLOGY AND BIOENGINEERING - COMBINATORIAL CHEMISTRY, vol. 83, no. 4, 20 August 2003 (2003-08-20), pages 416-427, XP002321671 NEW YORK, NY, US

<110> Samsung Electronics Co., Ltd.
<120> Method of concentrating and disrupting cells or viruses
<130> EP46685FZ106pau
<140> not yet assigned
   <141> herewith
<150> KR 10-2006-0031929
   <151> 2006-04-07
<160> 3
<170> KopatentIn 1.71
<210> 1
   <211> 18
   <212> DNA
   <213> Artificial Sequence
<220>
   <223> forward primer
<400> 1
   tgtatgaaga aggcttcg 18
<210> 2
   <211> 19
   <212> DNA
   <213> Artificial Sequence
<220>
   <223> reverse primer
<400> 2
   aaaggtatta actttactc 19
<210> 3
   <211> 68
   <212> DNA
   <213> Artificial Sequence
<220>
   <223> TagMan probe
<400> 3
   tgtatgaaga aggcttcggg ttgtaaagta ctttcagcgg ggaggaaggg agtaaagtta 60
   ataccttt 68

## Description

### BACKGROUND OF THE INVENTION

### 1. Field of the Invention

The present invention relates to a method for target cell separation and rapid nucleic acid isolation.

### 2. Description of the Related Art

Generally, molecular diagnosis of specific pathogenic bacteria is performed in four steps: cell lysis, DNA isolation, DNA amplification and DNA detection. Efficient extraction of DNA from cells is required in many applications and is essential in the molecular diagnosis, in particular, identification and quantification of pathogenic bacteria. Molecular diagnosis is generally performed by DNA amplification after DNA extraction. DNA amplification includes a polymerase chain reaction (PCR), a ligase chain reaction, stranded-displacement amplification, nucleic acid-based amplification, a repair chain reaction, a helicase chain reaction, QB replicase amplification, and ligation activated transcription.

Methods of isolating DNA from cells are performed using materials that have a proclivity of binding to DNA. Examples of materials for DNA isolation include silica, glass fiber, anion exchange resin and magnetic beads (Rudi, K. et al., Biotechniqures 22, 506-511 (1997); and Deggerdal, A. et al., Biotechniqures 22, 554-557 (1997)). To avoid manual operation and eliminate errors made by the operator, several automatic machines have been developed for high-throughput DNA extraction.

Cell lysis is conventionally performed using a mechanical, chemical, thermal, electrical, ultrasonic or microwave method (Michael T. Taylor et al., Anal. Chem., 73, 492-496 (2001)).

A laser has many advantages in the disruption of cells and can be readily applied to Lab-On-a-Chip (LOC) (Huaina Li et al., Anal Chem., 73, 4625-4631 (2001)).

U.S. Patent Publication No. 2003/96429 A1 discloses a laser-induced cell lysis system. However, this publication discloses a cell lysis system using only a laser, not mentioning that cell lysis is performed using magnetic beads and a laser according to the present invention.

Pitsillides, Costas M. et al.: "Selective cell targeting with light-absorbing microparticles and nanoparticles", Biophysical Journal, vol. 84, no. 6, pages 4023-4032, relates to a method for selected cell targeting based on the use of lidht-absorbing microparticles conjugated to antibodies, which kills cells selectively upon exposure to short laser pulses. Selective energy deposition for killing cells is accomplished by using laser pulses at a wavelength that is strongly absorbed by the particles but not by cells and with a pulse duration that is sufficiently short to minimize heat flow away from the absorbing particles, a condition known as thermal confinement.

EP 1 650 297 A2 discloses a method for concentrating and disrupting cells or viruses, the method including introducing magnetic beads into a cell-containing or virus-containing solution to concentrate the cells or viruses onto the magnetic beads; and irradiating a laser beam onto the magnetic beads to disrupt the cells or viruses

EP-A-0 605 003 describes an assay method for detecting the presence of bacteria, wherein the bacteria cells are selectively captured and removed from a sample by use of an antibody bound to magnetic beads. Thereafter, the captured cells are spread on a medium to form colonies and in the presence of colony material from the colonies of the bacteria of interest is detected by use of various procedures, including DNA or RNA probes, PCR techniques and labelled antibodies.

HOFMANN, O. et al.: "LASER BASED DISRUPTION OF BACILLUS SPORES ON A MICROCHIP" SPECIAL PUBLCIATION - ROYAL SOCIETY OF CHEMISTRY, LONDON, GB, 2005, pages 258-260 relates to laser based disruption of bacillus spores on a microchip, wherein the spores are mixed with the laser light-absorbing matrix and the mixture is then co-crystallized into nanovials. The sample zone of co-cystallized spores and matrix is irradiated with pulsed UV laser light effecting a disintegration of the matrix and concomitant disruption of the outer spore layer.

Generally, applying a method for target cell separation using antibody-coated magnetic beads that are commercially available, target cells can be separated from complex fluids such as whole blood. However, in subsequent processes, lysing nucleic acid is complicated, time-consuming, and requires various kinds of buffer solutions and thus is not suitable for a Lab-On-a-Chip.

Therefore, the inventors of the present invention studied the problems of the prior art, and confirmed that target cell separation/concentration and nucleic acid isolation could be rapidly performed such that after target cell separation/concentration using antibody or affinity binding-based magnetic beads, the magnetic bead-cell complex solution could be directly irradiated by a laser beam, and finally a nucleic acid could be separated.

### SUMMARY OF THE INVENTION

The present invention provides a method for rapid nucleic acid isolation using antibody or affinity binding-based magnetic beads that implemented on a Lab-On-a-Chip.

According to an aspect of the present invention, there is provided a method of according to claim 1.

### BRIEF DESCRIPTION OF THE DRAWINGS

The above and other features and advantages of the present invention will become more apparent by describing in detail exemplary embodiments thereof with reference to the attached drawings in which:
FIG. 1 is a schematic diagram of a cell lysis system using a laser and micro magnetic beads in a microchip.
FIG. 2A is a graph showing the results of real time PCR using a conventional method devoid of a cell concentration step and a method according to an embodiment of the present invention including cell concentration,
FIG. 2B represents the real time PCR results of FIG. 2A in terms of Rn values; and
FIG. 3 is a graph showing results of real time PCR performed after separating/concentrating *E*. *coli* resuspended in a phosphate-buffered saline (PBS) and a whole blood sample spiked with *E*, *coli.*

### DETAILED DESCRIPTION OF THE INVENTION

Hereinafter, the present invention will be described in more detail by explaining embodiments of the invention with reference to the attached drawings.

The present invention provides a method of isolating nucleic acid from concentrated and disrupted cells or viruses, the method comprising: introducing magnetic beads into a cell-containing or virus-containing solution to concentrate the cells or viruses onto the magnetic beads; irradiating a laser beam onto the magnetic beads to disrupt the cells or viruses; attaching denatured proteins and cell debris to the magnetic beads, while the DNA remains in solution; and separating the DNA by removing the magnetic beads by gravity or magnetic force, wherein the surface of the magnetic beads is treated with an antibody or metal oxide that has an affinity to target cells or viruses, wherein the metal oxide is selected from the group consisting of Al₂O₃, TiO₂, Ta₂O₃ and HfO₂.

In the method according to an embodiment of the present invention, target cells or viruses can be separated and concentrated from complex fluids, such as whole blood, saliva, urine and the like, preferably using antibody-coated magnetic beads that improve target cell separation. After concentrating the target cells onto the magnetic beads, a laser beam is directly irradiated thereto, and finally nucleic acids are rapidly isolated, and thus implementation on a Lab-On-a-Chip is possible. For example, when an antibody that specifically binds to biotin-bound cells or viruses is reacted with magnetic beads to which streptavidin is attached, the antibody is bound to the magnetic beads by a specific affinity binding between streptavidin and biotin. Then, a cell-containing or virus-containing sample is contacted with the bead comprising the antibody. As a result, the antibody specifically binds to specific cells or viruses so that these specific cells or viruses are concentrated. Therefore, if various kinds of antibodies are used, the designated target cells or viruses can be concentrated. Conventionally, cell lysis of concentrated cells or viruses is performed using a Boom method or the like, but this method is complicated and time-consuming. However, in the method according to the current embodiment of the present invention, nucleic acids can be rapidly and economically isolated since the magnetic beads used for cell or virus separation are also used for cell disruption. In particular, when a laser beam is irradiated onto cells or viruses bound to magnetic beads, the cells or viruses can be rapidly disrupted by the laser ablation phenomenon.

In the method according to the the present invention, the surface of the magnetic beads is treated with an antibody or metal oxide that has an affinity to target cells or viruses. The surface of the magnetic beads may be treated with an antibody that specifically binds the target cells or viruses. Since only desired specific cells or viruses are selectively concentrated by binding to the antibody, the method is useful in case of detecting cells or viruses that having a very low concentration in a sample solution. Magnetic beads, to which an antibody that can specifically bind to cells or viruses is bound, are commercially available from Invitrogen, Qiagen and the like. Examples thereof include Dynabeads® Genomic DNA Blood (Invitrogen), Dynabeads® anti-E. coli O157 (Invitrogen), CELLection^{™} Biotin Binder Kit (Invitrogen), MagAttract Virus Min M48 Kit (Qiagen) and the like. Using the magnetic beads to which the specific antibody is bound, *Diphtheria toxin, Enterococcus faecium, Helicobacter pylori, HBV, HCV, HIV, Influenza A, Influenza B, Listeria, Mycoplasma pneumoniae, Pseudomonas sp., Rubella virus, Rotavirus* and the like can be separated.

In the method according to the current embodiment of the present invention, a process step of vibrating the magnetic beads can be additionally included in a process of concentrating the cells or viruses. When the magnetic beads are vibrated, the magnetic beads are more likely to contact cells or viruses compared to cell concentration in which the magnetic beads are not vibrated. Thus, cells or viruses can be more efficiently concentrated onto the magnetic beads when the magnetic beads are vibrated. Magnetic beads can be vibrated by means of a vibrator such as a sonicator, a vibrator using a magnetic field, a vibrator using an electric field, or a mechanical vibrator such as a vortex or a piezoelectric material.

In the method according to the present invention, the metal oxide can be Al₂O₃, TiO₂, Ta₂O₃, and HfO₂. The metal oxide may be preferably Al₂O₃ or TiO₂, and more preferably Al₂O₃. Deposition of the metal oxide onto the beads can be performed using a physical vapor deposition (PVD) method, an atomic layer deposition (ALD) method, a sol-gel method or the like. A method of depositing the metal oxide onto the surface of magnetic beads is a known technique, and generally is performed using a PVD method, an ALD method, a sol-gel method or the like:

PVD is a method that is used in thin film formation, and has recently been favored as a means of surface curing, since a thin film can be relatively simply obtained by low temperature treatment that cannot be performed using other methods. Examples of PVD methods include an evaporation deposition method that does not use ions, a sputtering method that uses ions, an ion plating method, an ion implantation method, an ion beam mixing method and the like. In an ALD method, molecules are adsorbed onto a wafer surface using a chemically sticking property and then substituted. Since adsorption and substitution are alternatingly performed, ultrafine layer-by ultrafine layer deposition is possible, and an oxide and a metal thin film can be stacked as thin as possible. The sol-gel method is a method of preparing a metal oxide having a colloid form through hydrolysis reaction of a metal halide or alkoxide and is a method representative of preparing a titanium dioxide (Ti02) coating.

When target cells or viruses are bound to magnetic beads, a laser beam is irradiated to a solution containing magnetic beads and the magnetic beads cause an ablation by the laser, due to which shockwaves, vapor pressure and heat are transferred to the cell surface. At this time, physical shocks are also applied to the cell surface. The magnetic beads heated by the laser raise the temperature of the aqueous solution and directly disrupt the cells. The magnetic beads in the aqueous solution, however, do not simply act as a heat conductor but apply thermal, mechanical and physical shocks to the cell surface, thereby efficiently disrupting the cell surface.

When a laser beam is irradiated onto a cell solution devoid of magnetic beads, efficient cell lysis does not occur. The result of an experiment using *E*. *coli* placed in a very clear solution, it is confirmed that, when irradiating only a laser beam onto said clear solution, low cell lysis efficiency is obtained. A DNA concentration of 3.77 ng/µl was measured after irradiating a laser beam for 150 seconds because laser energy is not effectively transferred to cells without magnetic bead. Compared thereto, a DNA concentration of 6.15 ng/µl was measured after boiling cells at 95°C for 5 minutes is.

The rapid cell lysis using a laser and magnetic beads is performed by heating and laser ablation in a liquid medium. The laser treatment in combination with the micro magnetic beads converts the heat source into physical and mechanical shocks of highly heated magnetic beads to improve cell lysis. Currently, small size, high power laser diodes are rapidly being developed and a very small cell lysis apparatus using the same will be capable of being installed on a Lap-on-a-Chip (LOC). Moreover, the laser can concentrate the power and energy onto a specific region on a chip directly or by means of an optical fiber, mirror or lens.

The best advantage of the magnetic beads is that the number of DNA isolation steps can be reduced because the cell lysis by means of the micro magnetic beads in combination with laser irradiation results in the denaturation of proteins. The denatured proteins and cell debris are attached to the magnetic beads, while the DNA remains in solution. Thus, the denatured proteins and cell debris can be separated from the DNA when removing the magnetic beads by gravity or magnetic force. As a result, a detection limit is lowered, DNA extraction time is significantly shortened due to an omission of one step in the DNA extraction process, and polymerase chain reaction (PCR) analysis results are significantly improved due to an increase in the signal amplitude. The total time required for disrupting a cell using the micro magnetic beads and laser irradiation is only 40 seconds.

Laser ablation refers to a phenomenon that occurs in materials exposed to a laser beam. Laser ablation rapidly raises the temperature of a material surface from several hundred to several thousand degrees. If the temperature of the material surface is raised to the evaporation point of the material or higher, the saturated vapor pressure on the surface rapidly increases resulting in an evaporation of the liquid phase material. The saturated vapor pressure is expressed as a function of temperature by the Clausius-Clapeyron equation, and is usually raised to several tens of atmospheres or more in the case of a high power pulse laser process. Pressure applied to a material surface by vapor is referred to as "repulsive pressure" and the magnitude of the repulsive pressure is about 0.56 Pₛₐₜ where Pₛₐₜ denotes a vapor pressure.

A shockwave is generated in a process using a laser with very large instantaneous intensity, such as a pulse laser. The vapor generated on the surface of a material heated to its evaporation point or higher for a short time ranging from several nanoseconds to several tens of nanoseconds is increased to a pressure of several atmospheres to several tens of atmospheres and forms shockwaves while expanding into the surrounding medium. Due to the very high pressure, the expanding vapor applies about 0.56 Pₛ (where Pₛ denotes a saturated vapor pressure in the surface) to a material.

In the method according to the current embodiment of the present invention, the laser can be a pulse laser or continuous wave (CW) laser so that a laser pulse or a continuous laser wave, respectively, can be irradiated onto the magnetic beads. If the laser power is too low, laser ablation cannot effectively occur. The laser power is 10 mW or more for the CW laser and 1 mJ/pulse or more for the pulse laser. Preferably, the laser power for the pulse laser is 3 mJ/pulse or more and 100 mW or more for the CW laser. This is because not enough energy is transferred to disrupt the cells, when the laser power for the CW laser is less than 10 mW and the laser power for the pulse laser is less than 1 mJ/pulse.

In the method according to the current embodiment of the present invention, the laser beam should be generated as light having a specific wavelength range
where the magnetic beads can absorb the energy of the laser beam. The laser beam may be generated as light having a wavelength of above 400 nm, and preferably having a wavelength from 750 nm to 1,300 nm. This is because DNA is denatured or damaged when irradiated by light having a wavelength of less than 400 nm. The laser beam can also be generated comprising light having at least one wavelength range. That is, the laser light can have one wavelength or at least two different wavelengths within the above range.

In the method according to the current embodiment of the present invention, the diameter of the magnetic beads is preferably from 50 nm to 1,000 µm, and more preferably from 1 µm to 50 µm. When the diameter of the magnetic beads is less than 50 nm, physical and mechanical shocks are insufficient to cause cell lysis. When the diameter of the magnetic beads is greater than 1,000 µm, the beads are not suitable for Lab-On-a-Chip (LOC). The magnetic beads can also be a mixture of beads with at least two sizes. That is, the magnetic beads can be all the same size or be a mixture of beads with different sizes.

In the method according to the current embodiment of the present invention, the magnetic beads can be composed of any magnetized material. In particular, the magnetic beads may include at least one material selected from the group consisting of ferromagnetic Fe, Ni, Cr and oxides thereof.

In the method according to the current embodiment of the present invention, the magnetic beads can be polymers, organic materials, silicon or glass coated with a ferromagnetic metal.

In the method according to the current embodiment of the present invention, a solution containing magnetic beads may have a pH of 6-9. When the pH of the solution is beyond this range, the efficiency of DNA amplification decreases after cell lysis.

In the method according to the current embodiment of the present invention, the solution can be selected from the group consisting of saliva, urine, blood, serum and cell culture. The solution can be any solution having nucleic acids, such as animal cells, plant cells, bacteria, viruses, phage and the like.

Hereinafter, the present invention will be described in further detail with reference to the following examples. These examples are for illustrative purposes only and are not intended to limit the scope of the present invention.

### Example 1: E. coli cell lysis using the method of the present invention

### 1) E. coli cell preparation

*E. coli* K12 (ATCC 25404) as a bacterial cell was cultured in ATCC media 294, and then the bacterial cells were harvested by centrifugation and washed twice with 3 ml of phosphate-buffered saline (PBS). Subsequently the bacterial cells were resuspended in PBS (cell concentration: 1×10⁹ cells/ml). The obtained cells suspension was diluted in PBS to a final cell concentration of 1×10⁷ cells/ml.

### 2) Bead washing

Dynabeads® M-280 Streptavidin having a diameter of 2.8 µm, in which streptavidin was attached to the beads, were used as beads, and a bead solution was mixed to prepare a homogeneous solution. 100 µl of the prepared solution was placed in a tube, and left settle for two minutes in a magnetic field. The supernatant of the resulting solution was removed with a pipet. The tube was taken out of the magnetic field, and 100 µl of a buffer solution 1 (PBS containing 0.1 % of BSA, pH 7.4) was added to and mixed with the solution in the tube. Again, the tube was placed in the magnetic field and left to settle for two minutes. The supernatant of the resulting solution was removed with a pipet. The tube was taken out of the magnetic field, and 100 µl of a buffer solution 1 (PBS containing 0.1 % BSA, pH 7.4) was added to and mixed with the solution in the tube.

### 3) Pre-coating of beads using antibody

10 µl of *E*. *coli* antibody (virostat: 1007 biotin conjugate, 4-5 mg/ml) was placed in and mixed with the prepared bead solution. The solution was mixed by inverting the tube many times, and the mixture was incubated at room temperature for 30 minutes. The beads were collected for 1 minute using a magnet and the supernatant of the resulting solution was removed. 1 ml of a washing buffer solution (PBS containing 1 % BSA, pH 7.4) was added thereto and mixed by inverting the tube many times. The beads were collected for 1 minute using a magnet and the supernatant of the resulting solution was removed. 100 µl of a buffer solution 1 (PBS containing 0.1 % BSA, pH 7.4) was added thereto.

### 4) E. coli cell separation

100 µl of the *E*. *coli* K12 cell suspension prepared in Example 1-(1) and 100 µl of a bead solution containing beads to which the E. coli antibody was bound, prepared in Example 1-(3), were mixed. After mixing the two media by inverting with the tube many times, the mixture was incubated at room temperature for 20 minutes. The beads were collected for 1 minute using a magnet and the supernatant of the resulting solution was removed. 100 µl of a washing buffer solution was added thereto and mixed by inverting the tube many time. The beads were collected using a magnet and the supernatant of the resulting solution was removed. 4 µl of a buffer solution 1 (PBS, pH 7.4) was added thereto.

### 5) Cell lysis

4 µl of the solution obtained in Example 1-(4), said solution containing separated *E. coli* bound to the antibody coated on the bead, was injected into a vial located in a vial guide (AMITECH, Korea). 808 nm, 1 W laser beam (HLU25F100-808, LIMO, Germany) was applied to the mixture for 30 seconds for disrupting cells while stirring the vial by vortexing. (see Korean Patent Application No. 2005-78886)

### Example 2: Bacteria cell capture efficiency

The capture efficiency of *E. coli* K12 cell with respect to Dynabeads® M-280 Streptavidin to which *E. coli* antibody (virostat: 1007 biotin conjugate, 4-5 mg/ml) was bound, prepared in Example 1-(3), was determined. Three kinds of *E*. *coli* K12 suspensions having concentrations of 6.00E+09 cells/ml, 6.00E+08 cells/ml and 6.00E+07 cells/ml were used. Binding the *E*. *coli* cells to the beads was performed as described in above Example 1-(4). The number of *E. coli* cells in the sample suspension, the removed binding buffer and the removed washing buffer was counted using 3M colony count paper. The capture efficiency of *E*. *coli* K12 cell bound to the magnetic beads is shown in Table 1 below.

**Table 1**

| Sample Concentration (cells/ml) | Injection (100 µl) | Capture Efficiency (%) (N=4) (I-B-W)/I*100 | Coefficient of Variation (%) |
|---|---|---|---|
| 6.00E+09 | 6.00E+08 | 93.4 | 0.2 |
| 6.00E+08 | 6.00E+07 | 87.0 | 0.9 |
| 6.00E+07 | 6.00E+06 | 92.5 | 1.6 |

In Table 1, the column injection (100 µl) refers to the amount of cell introduced upon injecting 100 µl of the corresponding sample concentration. In Table 1, I refers to the number of *E*. *coli* cells in the injected solution, B refers to the number of *E. coli* cells remaining in a binding solution, and W represents the number of *E*. *coli* cells remaining in a washing buffer solution. Capture Efficiency (CE) is represented by the formula CE = ((I-B-W)/I*100). As can be seen in Table 1, *E*. *coli* cell capture efficiency is as high as about 90 %. Accordingly, when the method according to the current embodiment of the present invention is used, *E. coli* cells can be captured at a very high efficiency, and thus the method according to the current embodiment of the present invention is very useful for samples having a very low cell concentration. The coefficient of variation (%) is determined as (standard deviation/mean)*100

### Example 3: Effects of PCR inhibition by magnetic beads

When 250 mg/ml of Dynabeads® M-280 Streptavidin according to an embodiment of the present invention was used, the capture efficiency was about 90 % (see Example 2). In an experiment of cell capture using beads, 100 µl of a bead solution having a concentration of 10 mg beads/ml was reacted with 100 µl of *E*. *coli,* and the product was washed and the washing buffer was removed. Then the resulting product was eluted in 4 µl of an elution solution. As a result, the concentration of the bead was 250 mg/ml in the elution solution. The bead concentration was 25 times as high as the bead concentration of Dynabeads® MyOne^{™} Carboxylic Acid (DYNAL, Norway) (10 mg/ml) in which the bead is used as a material that generates cell lysis without having a cell concentration function. Therefore, whether PCR inhibition occurs when the amount of beads is increased was determined.

As in Example 2, 6×10⁹ cells/ml, 6×10⁸ cells/ml and 6×10⁷ cells/ml were used as a concentration of *E*. *coli* K12. Cells in a cell and bead mixture comprising 10 % of beads and 90 % of a cell solution were lysed using laser irradiation, and then TaqMan real time PCR was performed. PCR was performed by completely denaturing DNAs in a PCR mixture (75 mM Tris-HCl (pH 9.0), 15 mM (NH₄)₂SO₄, 5 mM MgCl₂, 1 mg/ml BSA, 250 µM dNTP mixture, 1 µM PCR primer (SEQ ID NO: 1 and SEQ ID NO: 2) and 0.4 µM TaqMan probe (FAM-5'-TGTATGAAGAAGGCTTCGGGTTGTAAAGTACTTTCAGCGGGGAGGAAG GGAGTAAAGTTAATACCTTT-3'-TAMRA: SEQ ID NO: 3) using GeneSpector® Micro PCR (SAIT, Korea) at a temperature of 95°C for 1 minute, and then 40 cycles (10 seconds at 95°C, 10 seconds at 50°C and 10 seconds at 72°C) were performed. The results of PCR in term of the crossing point (Cp) of the PCR product are shown in Table 2. Cp refers to the cycle number in which a detectable fluorescence signal occurs in a real time PCR. That is, as an initial DNA concentration is higher, it is possible for a fluorescence signal to be detected at a lower Cp, and the lower the initial DNA concentration is, the higher is the Cp. Cp is also related to DNA purification, that is, the higher DNA purity is, the lower is the Cp, and the lower DNA purity is, the higher is the Cp. Accordingly, as Cp is lower, DNA in a solution has a more purified form.

**Table 2**

| Injected cell (cells/ml) | Myone bead (10.0 mg/ml) | M-280 Streptavidine bead (mg/ml) | | | |
|---|---|---|---|---|---|
| | | 12.5 | 25.0 | 50.0 | 250.0 |
| 6.00E+09 | 22.90 | 22.36 | 22.52 | 22.04 | 22.53 |
| 6.00E+08 | 24.21 | 24.53 | 23.87 | 24.22 | 23.67 |
| 6.00E+07 | 28.36 | 25.78 | 25.11 | 25.99 | 27.40 |

As can be seen in Table 2, even when an amount of beads used in the present invention that is 25 times the amount of Dynabeads® MyOne^{™} Carboxylic Acid (DYNAL, Norway), there was little impact on the PCR. Therefore, when the method according to the current embodiment of the present invention is used, the cell capture efficiency is as high as about 90%, and magnetic beads used in nucleic acid binding and lysis barely influence nucleic acid amplification of the DNA released from the captured cells Thus, magnetic beads according to an embodiment of the present invention are very useful for capturing cells and isolating nucleic acids.

### Example 4: Effects of cell concentration using the method according to the current embodiment of the present invention

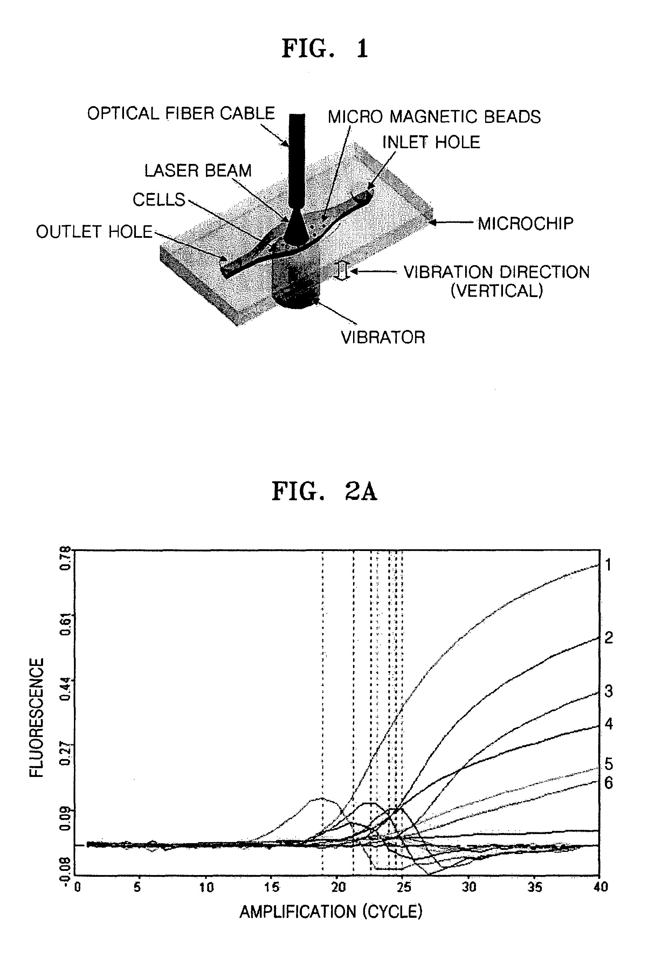

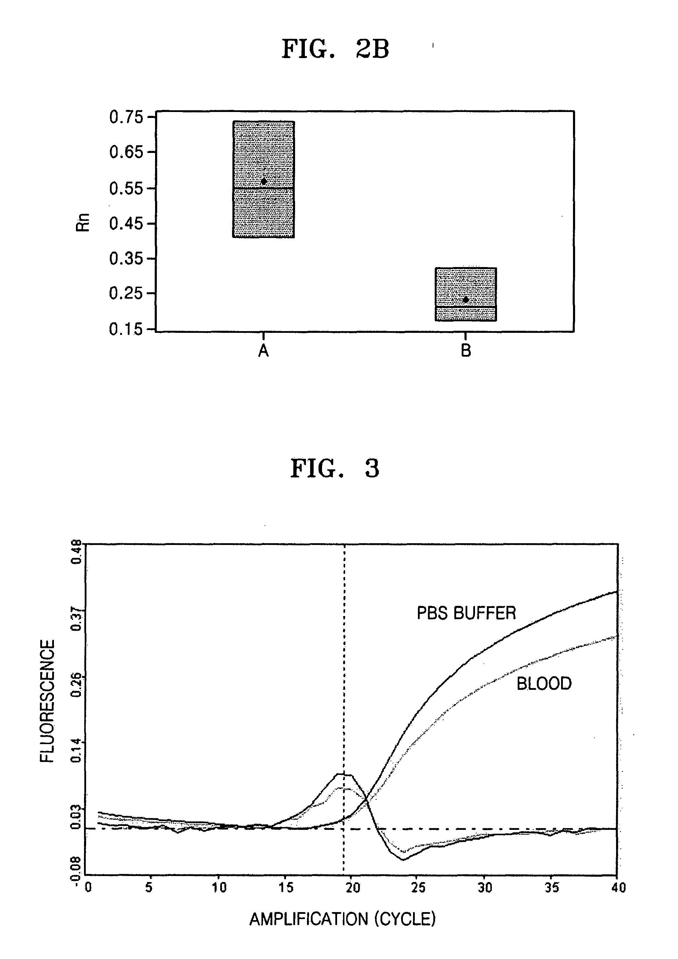

Compared with a conventional method of mixing a micro magnetic bead (Dynabeads® MyOne^{™} Carboxylic Acid, DYNAL, Norway), cell capture efficiency using the method according to the current embodiment of the present invention was determined. An experiment similar to Example 1 was performed. The results in terms of the measured Cp and Rn of the real time PCR product are shown in FIGS. 2A and 2B. FIG. 2A is a graph showing the results of a real time PCR using a conventional method (Korean Patent Application No. 2005-78886) excluding a step of cell concentration and the method according to the current embodiment of the present invention including a cell concentration step prior to cell lysis. In FIG. 2A, the method according to the current embodiment of the present invention including a cell concentration step corresponds to "1 ", "2" and "3" represented on the right side of the graph. Initial cell solutions having cell concentration of 10⁹ cells/ml, 10⁸ cells/ml and 10⁷ cells/ml were used for "1", "2" and "3", respectively. The conventional method (Korean Patent Application No. 2005-78886) excluding a cell concentration step corresponds to "4", "5" and "6" represented on the right side of the graph. Initial cell solutions having cell concentration of 10⁹ cells/ml, 10⁸ cells/ml and 10⁷ cells/ml were used for "4", "5" and "6", respectively. In FIG. 2B, the real time PCR results of FIG. 2A are represented as Rn-values. Rn is related to the final concentration of the PCR product obtained, and the higher Rn is, the higher is the concentration of a PCR product. In FIG. 2B, A refers to the PCR results using the method according to the current embodiment of the present invention including a cell concentration step, and B represents the PCR results using the conventional method. Cp is related to an initial PCR template concentration, and the lower the Cp is, the higher is the initial concentration. If the PCR efficiency is 100 %, a difference (Δ) in the Cp of 3.3 denotes a 10 times difference in the initial template concentration. Results of FIG. 2 are shown in Table 3 below.

**Table 3**

| Comparison method | Cell concentration (cells/ml) | Cp | Rn | ΔCp | ΔRn |
|---|---|---|---|---|---|
| Korean Patent Application No. 2005-78886 (no cell concentration step) | 1.00E+09 | 21.25 | 0.32 | | |
| | 1.00E+08 | 23.04 | 0.21 | | |
| | 1.00E+07 | 24.96 | 0.17 | | |
| The present invention (including cell concentration step) | 1.00E+09 | 18.88 | 0.74 | 2.37 | 0.43 |
| | 1.00E+08 | 22.57 | 0.55 | 0.47 | 0.34 |
| | 1.00E+07 | 24.51 | 0.41 | 0.45 | 0.23 |

As can be seen in FIG. 2 and Table 3, compared with a conventional method of mixing a micro magnetic bead (Dynabeads® MyOne^{™} Carboxylic Acid, DYNAL, Norway), cell capture efficiency using the method according to the current embodiment of the present invention is about 2-7 times higher than the cell capture efficiency using the conventional method. Accordingly, when the method according to the current embodiment of the present invention is used, cell concentration efficiency is very high compared with the conventional method.

### Example 5: Effects of cell concentration from whole blood using the method according to the current embodiment of the present invention

To selectively separate/concentrate *E*. *coli* from in human whole blood spiked with *E. coli* cells, contrary to concentrating cells in a phosphate-buffered saline, the method of Example 1 was performed. FIG. 3 is a graph showing the results of real time PCR performed after separating/concentrating *E*. *coli* in a phosphate buffer solution and in whole blood. Results of FIG. 3 are shown in Table 4 below.

**Table 4**

| Sample types | Cp | Rn | PCR product concentration (ng/ml) |
|---|---|---|---|
| Whole blood | 19.43 | 0.322 | 20.3 |
| PBS buffer | 19.40 | 0.400 | 17.9 |

As can be seen in FIG. 3 and Table 4, when whole blood was used as the sample, cell capture efficiency and PCR results were similar to the results obtained when a phosphate-buffered saline was used.

Therefore, even when real samples, such as blood, saliva, urine and the like, that cannot be directly applied to PCR are used, cell capture and PCR can be efficiently performed.

As described above, according to the method of the present invention, since the same commercially available magnetic beads are used in target cell separation and cell lysis, beads need not to be additionally added in a laser lysis process, and thus integration of a target cell separation, concentration, purification, and nucleic acid extraction process is can easily be achieved. Also, in a conventional method, blood acts as an inhibitor for PCR, so that blood samples cannot be directly used. However, according to the present invention, target cells in blood can be easily separated and concentrated using beads for target cell separation, and nucleic acid extraction can be easily integrated by irradiating a laser beam onto the same beads. Thus, target cells in blood can be lysed and processed by PCR only after cell separation and purification.

While the present invention has been particularly shown and described with reference to exemplary embodiments thereof, it will be understood by those of ordinary skill in the art that various changes in form and details may be made therein without departing from the spirit and scope of the present invention as defined by the following claims.
## Claims

1. A method for isolating nucleic acid from concentrated and disrupted cells or viruses, the method comprising:
introducing magnetic beads into a cell-containing or virus-containing solution to concentrate the cells or viruses onto the magnetic beads;
irradiating a laser beam onto the magnetic beads to disrupt the cells or viruses;
attaching denatured proteins and cell debris to the magnetic beads, while the DNA remains in solution; and
separating the DNA by removing the magnetic beads by gravity or magnetic force;
wherein the surface of the magnetic beads is treated with an antibody or metal oxide that has an affinity to target cells or viruses, wherein the metal oxide is selected from the group consisting of Al₂O₃, TiO₂, Ta₂O₃ and HfO₂.

2. The method of claim 1, wherein concentrating the cells or viruses onto the magnetic beads further comprises vibrating the magnetic beads.

3. The method of claim 1, wherein the laser beam is a laser pulse or a continuous laser wave.

4. The method of claim 3, wherein the power of the laser pulse is more than 1 mJ/pulse, and the power of the continuous laser wave is more than 10 mW.

5. The method of claim 4, wherein the power of the laser pulse is more than 3 mJ/pulse, and the power of the continuous laser wave is more than 100 mW.

6. The method of claim 1, wherein the light of the laser beam generated has a wavelength of above 400 nm.

7. The method of claim 6, wherein the light of the laser beam generated has a wavelength of 750 nm - 1,300 nm.

8. The method of claim 6, wherein the laser beam consists of light having at least one wavelength range.

9. The method of claim 1, wherein the magnetic beads have a size of 50 nm to 1,000 µm.

10. The method of claim 9, wherein the magnetic beads have a size of 1 µm to 50 µm.

11. The method of claim 9, wherein the magnetic beads are a mixture of beads having at least two sizes.

12. The method of claim 1, wherein the magnetic beads comprise at least one material selected from the group consisting of ferromagnetic Fe, Ni, Cr and oxides thereof.

13. The method of claim 1, wherein the magnetic beads are coated with ferromagnetic metals on polymers, organic materials, silicon, or glass.

14. The method of claim 1, wherein a solution containing the magnetic beads has a pH of 6-9.

15. The method of claim 1, wherein the solution is selected from the group consisting of saliva, urine, blood, serum and cell culture.

## Patentansprüche

1. Verfahren zum Isolieren von Nukleinsäure aus angereicherten und aufgeschlossenen Zellen oder Viren, wobei das Verfahren umfasst:
Einbringen von magnetischen Kügelchen in eine Lösung, die Zellen oder Viren enthält, um die Zellen oder Viren an den magnetischen Kügelchen anzureichern;
Strahlen eines Laserstrahles auf die magnetischen Kügelchen, um die Zellen oder Viren aufzuschließen;
Anlagern von denaturierten Proteinen oder Zellbruchstücken an die magnetischen Kügelchen, während die DNA in Lösung bleibt; und
Abtrennen der DNA durch Entfernen der magnetischen Kügelchen mittels Schwerkraft oder einer magnetischen Kraft;
wobei die Oberfläche von den magnetischen Kügelchen mit einem Antikörper oder einem Metalloxid, das eine Affinität zu einer Ziel-Zelle oder einem Ziel-Virus aufweist, behandelt ist, wobei das Metalloxid ausgewählt ist aus der Gruppe bestehend aus Al₂O₃, TiO₂, Ta₂O₃ und HfO₂.

2. Verfahren gemäß Anspruch 1, wobei das Anreichern der Zellen oder Viren an den magnetischen Kügelchen ferner das Schütteln der magnetischen Kügelchen umfasst.

3. Verfahren gemäß Anspruch 1, wobei der Laserstrahl ein Laserpuls oder eine Dauerstrich-Laserstrahlung ist.

4. Verfahren gemäß Anspruch 3, wobei die Leistung des Laserpulses größer als 1 mJ/Puls ist, und wobei die Leistung der Dauerstrich-Laserstrahlung größer als 10 mW ist.

5. Verfahren gemäß Anspruch 4, wobei die Leistung des Laserpulses größer als 3 mJ/Puls ist, und wobei die Leistung Leistung der Dauerstrich-Laserstrahlung größer als 100 mW ist.

6. Verfahren gemäß Anspruch 1, wobei das Licht des erzeugten Laserstrahls eine Wellenlänge von über 400 nm aufweist.

7. Verfahren gemäß Anspruch 6, wobei das Licht des erzeugten Laserstrahls eine Wellenlänge von 750 nm bis 1.300 nm aufweist.

8. Verfahren gemäß Anspruch 6, wobei der Laserstrahl aus Licht, das wenigstens einen Wellenlängenbereich aufweist, besteht.

9. Verfahren gemäß Anspruch 1, wobei die magnetischen Kügelchen eine Größe von 50 nm bis 1.000 µm aufweisen.

10. Verfahren gemäß Anspruch 9, wobei die magnetischen Kügelchen eine Größe von 1 µm bis 50 µm aufweisen.

11. Verfahren gemäß Anspruch 9, wobei die magnetischen Kügelchen ein Gemisch aus Kügelchen mit wenigstens zwei Größen sind.

12. Verfahren gemäß Anspruch 1, wobei die magnetischen Kügelchen wenigstens ein Material umfassen, das ausgewählt ist aus der Gruppe bestehend aus ferromagnetischem Fe, Ni, Cr und deren Oxiden.

13. Verfahren gemäß Anspruch 1, wobei die magnetischen Kügelchen mit einem ferromagnetischen Material auf einem Polymer, organischem Material, Silikon oder Glas beschichtet sind.

14. Verfahren gemäß Anspruch 1, wobei die Lösung, welche die magnetischen Kügelchen enthält, einen pH von 6 bis 9 aufweist.

15. Verfahren gemäß Anspruch 1, wobei die Lösung ausgewählt ist aus der Gruppe bestehend aus Speichelflüssigkeit, Urin, Blut, Serum und einer Zellkultur.

## Revendications

1. Procédé d'isolement d'acide nucléique à partir de virus ou de cellules concentrés et dissociés, ledit procédé consistant à :
introduire des billes magnétiques dans une solution contenant des cellules ou contenant des virus pour concentrer les cellules ou les virus sur les billes magnétiques ;
irradier les billes magnétiques avec un faisceau laser pour dissocier les cellules ou les virus ;
attacher des protéines dénaturées et des débris cellulaires aux billes magnétiques, tandis que l'ADN reste en solution ; et
séparer l'ADN par élimination des billes magnétiques sous l'effet de la gravité ou d'une force magnétique ;
où la surface des billes magnétiques est traitée avec un anticorps ou un oxyde métallique qui a une affinité pour les cellules ou virus cibles, l'oxyde métallique étant choisi dans le groupe constitué de Al₂O₃, TiO₂, Ta₂O₃ et HfO₂.

2. Procédé de la revendication 1, dans lequel la concentration des cellules ou des virus sur les billes magnétiques consiste en outre à faire vibrer les billes magnétiques.

3. Procédé de la revendication 1, dans lequel le faisceau laser est un laser à impulsions ou un laser à onde continue.

4. Procédé de la revendication 3, dans lequel l'énergie du laser à impulsions est supérieure à 1 mJ/impulsion et la puissance du laser à onde continue est supérieure à 10mW.

5. Procédé de la revendication 4, dans lequel l'énergie du laser à impulsions est supérieure à 3 mJ/impulsion et la puissance du laser à onde continue est supérieure à 100 mW.

6. Procédé de la revendication 1, dans lequel la lumière générée par le faisceau laser a une longueur d'onde supérieure à 400 nm.

7. Procédé de la revendication 6, dans lequel la lumière générée par le faisceau laser a une longueur d'onde de 750 nm - 1300 nm.

8. Procédé de la revendication 6, dans lequel le faisceau laser consiste en une lumière ayant au moins une gamme de longueurs d'onde.

9. Procédé de la revendication 1, dans lequel les billes magnétiques ont une taille de 50 nm à 1000 µm.

10. Procédé de la revendication 9, dans lequel les billes magnétiques ont une taille de 1 µm à 50 µm.

11. Procédé de la revendication 9, dans lequel les billes magnétiques sont un mélange de billes ayant au moins deux tailles.

12. Procédé de la revendication 1, dans lequel les billes magnétiques comprennent au moins un matériau choisi dans le groupe constitué des Fe, Ni, Cr ferromagnétiques, et des oxydes de ceux-ci.

13. Procédé de la revendication 1, dans lequel les billes magnétiques sont revêtues de métaux ferromagnétiques sur des polymères, matériaux organiques, silicium ou verre.

14. Procédé de la revendication 1, dans lequel une solution contenant les billes magnétiques a un pH de 6-9.

15. Procédé de la revendication 1, dans lequel la solution est choisie dans le groupe constitué de la salive, de l'urine, du sang, du sérum et d'une culture cellulaire.
